# DEMANDE DE BREVET EUROPEEN

(11) **EP 0 983 770 A1**
(43) Date de publication de la demande: **08.03.2000**
(21) Numéro de dépôt: 98402094.1
(22) Date de dépôt: 21.08.1998
(51) Int. Cl.: A61M 3/02, A61J 1/00

(54) **Tubulure d'alimentation entre une source de sérum physiologique et une cavité d'un organisme vivant**

(71) Demandeur: Guignard, Claude, 01630 Saint-Genis-Pouilly (FR)
(72) Inventeur: Guignard, Claude, 01630 Saint-Genis-Pouilly (FR)
(74) Mandataire: Savoye, Jean-Paul

(57) **Abrégé**

Cette tubulure d'alimentation entre une source de sérum physiologique (3) et une cavité (2) d'un organisme vivant comprend, en amont de son extrémité aval de distribution dudit sérum dans ladite cavité (2), une partie renflée (10) à paroi souple, une valve anti-retour (11) étant disposée en amont de cette partie renflée (10) pour ne permettre audit sérum physiologique de ne s'écouler que dans le sens allant de ladite source (3) à ladite cavité (2).

## Description

La présente invention se rapporte à une tubulure d'alimentation entre une source de sérum physiologique et une cavité d'un organisme vivant.

Le principe de la perfusion sous gravité est, de nos jours, largement utilisé en chirurgie endoscopique. Lorsque les pressions de l'ordre de 16 kPa (120 mm/Hg) sont nécessaires en permanence pour l'hémostase, la poche de sérum physiologique doit être disposée à une hauteur constante minimum de 1,6 mètres au-dessus de la cavité du corps de l'organisme vivant à irriguer. Compte tenu du fait que cette dernière est située environ à 1 m au-dessus du sol, la hauteur sous plafond est juste suffisante pour une intervention classique arthroscopique du type ménisectomie.

Il peut arriver qu'il soit nécessaire d'augmenter ponctuellement cette pression parce que le liquide dans le champ opératoire se trouble ou parce qu'il est nécessaire d'évacuer un débris qui réduit ou arrête le débit de liquide dans la tubulure d'évacuation du liquide de la cavité du corps. On peut également avoir besoin d'augmenter la pression pour faire une hémostase. Pour obtenir cette augmentation ponctuelle de pression, il faut alors comprimer les parois souples de la poche de sérum physiologique constituant la source de ce sérum pour accroître la pression du liquide dans la tubulure. Compte tenu de la hauteur à laquelle se trouve cette poche, le chirurgien qui opère ne peut pas le faire lui-même et doit avoir recours à un assistant de la salle d'opération, ce qui n'est évidemment ni pratique ni rapide.

Il existe différents moyens qui permettent de créer une pression autrement que par gravité et, par la même occasion, qui permettent de faire varier cette pression par une commande qui peut être actionnée par le chirurgien lui-même sans avoir à se déplacer. C'est le cas par exemple des système d'irrigation utilisant des pompes péristaltiques, avec lesquelles il s'agit d'accroître la vitesse de rotation pour augmenter le débit/pression du liquide dans la tubulure, cette commande étant généralement réalisée par le chirurgien par l'actionnement d'une pédale prévue à cet effet.

Ces dispositifs ont fait leurs preuves, mais coûtent relativement cher, de sorte que leur achat ne se justifie pas forcément pour des hôpitaux ou cliniques à faible capacité opératoire.

Ceci dit, même avec les appareils d'irrigation permettant au chirurgien de varier ponctuellement la pression en cas de besoin, celui-ci n'a pas de moyen de visualiser si la pression dans la cavité dans laquelle il opère est bien la pression affichée par le manomètre. En effet, cette pression est celle qui règne à l'endroit de la tubulure auquel est relié le manomètre. Or, il est possible qu'il y ait un écrasement local de la tubulure entre l'endroit de captage et le cavité à irriguer et il faudra un certain temps au chirurgien pour s'en apercevoir. Par ailleurs, dans le cas d'une arthroscopie du genou par exemple, le gonflement du genou varie d'un patient à l'autre en fonction de sa morphologie et du vieillissement des tissus, ne donnant de ce fait, ni une indication visuelle, ni une indication tactile fiable.

Le but de la présente invention est de remédier, au moins en partie, aux inconvénients susmentionnés.

A cet effet, l'objet de la présente invention est une tubulure du type susmentionné, telle que définie par la revendication 1

Grâce à la tubulure selon la présente invention, une pression momentanée sur la partie renflée permet d'augmenter ponctuellement la pression pour éclaircir le champ opératoire ou pour évacuer un débris obstruant la tubulure d'évacuation. Lorsque cette tubulure est utilisée aussi bien avec une irrigation par gravité que par une pompe ou tout autre moyen de créer une pression supérieure à la pression atmosphérique, la partie renflée à paroi souple permet de rendre visible le tait que la pression est effectivement transmise jusqu'à la cavité, puisqu'en cas contraire, la paroi souple de la partie renflée ne sera pas tendue. Le chirurgien dispose ainsi d'un témoin visuel.

Le dessin annexé illustre, schématiquement et à titre d'exemple, une forme d'exécution de la tubulure objet de la présente invention.
La figure 1 est une vue en élévation de cette forme d'exécution utilisée dans le cadre d'une endoscopie;
la figure 2 est une vue agrandie d'une portion de cette tubulure.

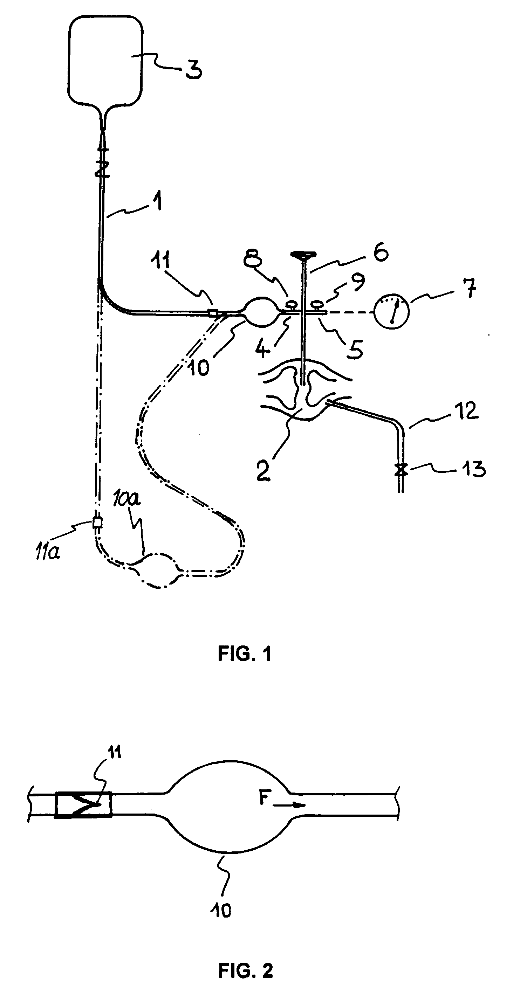

Dans le cas de la figure 1, une tubulure 1, de préférence en PVC selon la présente invention, est utilisée pour alimenter une cavité 2 d'un organisme vivant. Il peut s'agir par exemple de l'arthroscopie d'un genou. Une source d'alimentation en sérum physiologique, constituée par une poche souple 3 contenant une solution de NaCl en concentration physiologique, est connectée de façon étanche à une extrémité de la tubulure 1. La poche souple 3 est disposée à une certaine hauteur au-dessus de la cavité 2 à alimenter, cette hauteur étant choisie en fonction de la pression d'alimentation désiré. En pratique, la pression est limitée par la différence de hauteur entre le plafond de la salle d'opération et le patient.

L'autre extrémité de cette tubulure 1 est connectée de façon étanche, à l'une 4 des deux entrées latérales 4, 5 d'un trocart 6 dont une extrémité pénètre dans la cavité, tandis que l'autre extrémité sert à recevoir la caméra (non représentée) de l'endoscope. La seconde entrée larérale 5 du trocart 6 peut être, si nécessaire, connectée à un manomètre 7. Les deux entrées latérales 4, 5 du trocart 6 sont contrôlées chacune par un robinet 8, respectivement 9.

En amont de sa connexion avec le trocart 6, la tubulure 1 présente une partie renflée 10. La section de cette partie renflée 10 croit puis décroît progressivement, pour lui donner la forme d'un ballonnet dont le volume est choisi pour pouvoir être pris dans une main. Ce volume est compris entre 100 et 300 ml, idéalement entre 200 et 300 ml.

A l'extrémité d'entrée du ballonnet, c'est-à-dire l'extrémité amont dirigée vers la poche souple 3 contenant le liquide physiologique qui s'écoule vers la cavité 2, une valve anti-retour 11 (figure 2) contrôle l'écoulement du sérum physiologique et assure que celui-ci ne peut s'écouler que dans un sens, c'est-à-dire dans le sens de l'écoulement F du sérum physiologique, allant de la partie renflée 10 vers la cavité 2.

Pour permettre la circulation du liquide à travers la cavité 2, un conduit d'évacuation 12 contrôlé par un robinet 13 est connecté à la cavité 2.

Lorsque le chirurgien en cours d'intervention a besoin d'augmenter la pression momentanément, pour dégager le conduit d'écoulement obstrué par un débris, ou pour effectuer une hémostase, il saisit la partie renflée 10 et la comprime. La surpression ainsi créée provoque la fermeture de la valve anti-retour 11, de sorte que cette surpression règne dans la partie de la tubulure 1 située en aval de cette valve anti-retour 11. Lorsqu'elle sert à chasser un débris qui se trouve dans le conduit de sortie 12, le robinet 13 doit évidemment être ouvert. Lorsqu'elle sert à faire une hémostase dans la cavité 2, le robinet 13 contrôlant le conduit d'évacuation 12 doit au contraire être fermé.

Cette partie renflée 10 peut également servir à augmenter momentanément le débit, notamment après une hémostase, pour faire circuler un plus grand débit de liquide afin de rétablir sa transparence et d'éclaircir ainsi la zone d'intervention. Elle permet également de visualiser la pression qui règne dans la cavité 2, puisqu'elle perd immédiatement sa forme de ballonnet dès que la pression chute.

Bien entendu, la tubulure 11 selon la présente invention n'est pas limitée à une alimentation du sérum physiologique par gravité, comme dans l'exemple décrit, mais est également utilisable avec d'autres systèmes d'alimentation, tels que les alimentations par pompes péristaltiques ou tout autre système d'alimentation sous pression supérieure à la pression atmosphérique. Même si ces systèmes permettent en général une variation du débit ou de la pression, aucun d'eux ne fournit au chirurgien un témoin visuel lui permettant de constater que la cavité est bien sous pression. Il s'agit là d'une sécurité supplémentaire plus immédiate que la lecture d'un manomètre qui lui, sert à fixer la valeur de la pression. Mais si, par exemple la tubulure 11 est accidentellement pliée empêchant la pression de régner en aval, ceci peut être immédiatement constaté visuellement et/ou en palpant le ballonnet 10, beaucoup plus facilement que par la lecture du cadran d'un manomètre.

En variante, on pourrait aussi allonger la tubulure 1 et disposer la partie renflée 10 formant le ballonnet à une certaine distance de l'entrée 4 du trocart 6, par exemple 1 mètre, de manière à ce qu'il repose sur le sol, afin de permettre au chirurgien de l'actionner avec le pied.

Comme illustré à titre d'autre variante en traits mixtes sur la figure 1, on pourrait aussi avoir deux parties renflées 10 et 10a disposées le long de la tubulure 1, l'une 10 située vers l'entrée 4 du trocart 6, l'autre 10a à une plus grande distance en amont du premier, de manière à reposer sur le sol. La valve anti-retour 11a se situera alors en amont de la seconde partie renflée 10a.

## Revendications

1. Tubulure d'alimentation entre une source de sérum physiologique (3) et une cavité (2) d'un organisme vivant, caractérisée en ce qu'en amont de son extrémité aval de distribution dudit sérum dans ladite cavité (2), la tubulure (11) présente au moins une partie renflée (10) à paroi souple, une valve anti-retour (11) étant disposée en amont de cette partie renflée (10) pour ne permettre audit sérum physiologique de ne s'écouler que dans le sens allant de ladite source (3) à ladite cavité (2).

2. Tubulure selon la revendication 1, caractérisée en ce que ladite partie renflée (10) est constituée par un segment de ladite tubulure (11) réalisé en un matériau souple non extensible, dont la section augmente progressivement jusqu'à une valeur déterminée, à partir de chacune des extrémités dudit segment.

3. Tubulure selon l'une des revendications précédentes, caractérisée en ce que la longueur dudit segment formant ladite partie renflée (10) est comprise entre 5 et 10 cm et que son volume est d'au moins 100 ml.

4. Tubulure selon la revendication 4, caractérisée en ce que le volume de ladite partie renflée est de 200 ml à 300 ml.

5. Tubulure selon l'une des revendications précédentes. caractérisée en ce que ladite tubulure (1) et ladite partie renflée (10) sont formées d'une seule pièce.

6. Tubulure selon l'une des revendications précédentes, caractérisée en ce qu'elle comporte deux parties renflées (10, 10a), la valve anti-retour (11a) étant située en amont de la partie renflée (10a) la plus en amont le long de la tubulure (1).
